# EUROPEAN PATENT APPLICATION

(11) **EP 4 769 077 A1**
(43) Date of publication of application: **01.07.2026**
(21) Application number: 25201303.2
(22) Date of filing: 10.09.2025
(51) Int. Cl.: G06F 3/01

(54) **POSTURE TRACKING APPARATUS AND METHOD**

(30) Priority: 25.12.2024 US 202419001438
(71) Applicant: HTC Corporation, Taoyuan City 330, (TW)
(72) Inventor: YANG, WanLing, 330 Taoyuan City (TW); WANG, Sui-Hsien, 330 Taoyuan City (TW)
(74) Representative: Klang, Alexander H.

(57) **Abstract**

A posture tracking apparatus and method are provided. The apparatus captures an eye image of a user. The apparatus calculates an eye posture based on the eye image. The apparatus captures a reflected image from the eye image. The apparatus calculates a head posture of the user based on the reflected image and the eye posture.

## Description

### BACKGROUND

### Field of Invention

The present disclosure relates to posture tracking apparatus and method. More particularly, the present disclosure relates to posture tracking apparatus and method based on an eye image.

### Description of Related Art

In the existing technology, in order to track the head posture of a user and construct a scene, a head-mounted apparatus usually needs to rely on the image of the scene and the parameters of the camera itself (e.g., the relationship between multiple shooting angles, image deformation parameters) for calculation. On the other hand, in order to track the facial movements and/or eye movements of the user, the head-mounted apparatus also needs to be equipped with an image capture unit facing the user.

It can be seen from this that in order to achieve the functions above, the head-mounted apparatus needs to be equipped with multiple cameras facing different directions, resulting in adverse effects such as limited hardware specifications, increased manufacturing costs, and increased weight. In addition, due to privacy or confidentiality considerations, the camera used to capture the scene may capture images that the user does not want to be captured.

In view of this, how to provide a more convenient head posture tracking technology is the goal that the industry strives to work on.

### SUMMARY

The disclosure provides a posture tracking apparatus comprising a camera and a processor. The camera is configured to capture an eye image of a user. The processor is electrically connected to the camera and configured to execute the following operation: calculating an eye posture based on the eye image; capturing a reflected image from the eye image; and calculating a head posture of the user based on the reflected image and the eye posture.

The disclosure further provides a posture tracking method being adapted for use in an electronic apparatus, wherein the posture tracking method comprises the following steps: capturing an eye image of a user; calculating an eye posture based on the eye image; capturing a reflected image from the eye image; and calculating a head posture of the user based on the reflected image and the eye posture.

It is to be understood that both the foregoing general description and the following detailed description are by examples, and are intended to provide further explanation of the disclosure as claimed.

### BRIEF DESCRIPTION OF THE DRAWINGS

The disclosure can be more fully understood by reading the following detailed description of the embodiment, with reference made to the accompanying drawings as follows:
Fig. 1 is a schematic diagram illustrating an environment where a user wearing a head-mounted apparatus according to some embodiments of the present disclosure.
Fig. 2 is a schematic diagram illustrating a posture tracking apparatus according to a first embodiment of the present disclosure.
Fig. 3 is a schematic diagram illustrating the posture tracking apparatus capturing a reflected image from an eye image according to some embodiments of the present disclosure.
Fig. 4 is a flow diagram illustrating a posture tracking method according to a second embodiment of the present disclosure.

### DETAILED DESCRIPTION

Reference will now be made in detail to the present embodiments of the disclosure, examples of which are illustrated in the accompanying drawings. Wherever possible, the same reference numbers are used in the drawings and the description to refer to the same or like parts.

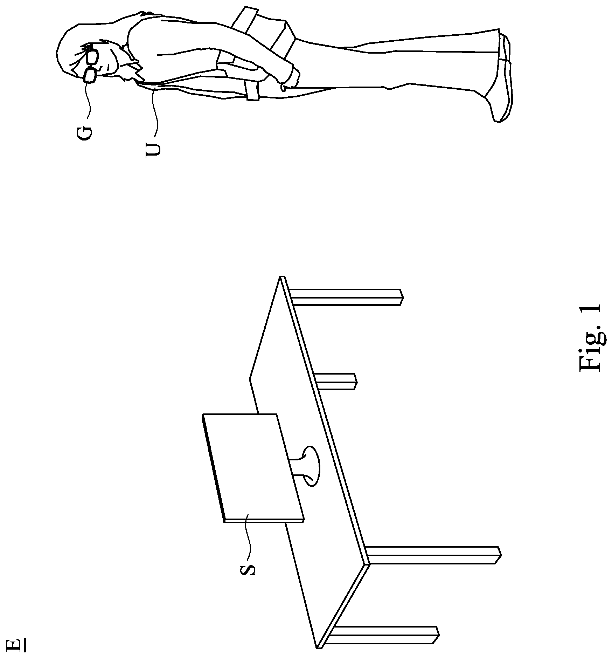

Please refer to Fig. 1, which is a schematic diagram illustrating an environment E where a user U wearing a head-mounted apparatus G according to some embodiments of the present disclosure. As shown in Fig. 1, the user U wears the head-mounted apparatus G. The environment E is where the user U operates the head-mounted apparatus G. There is a specific object S in the environment E, and the specific object S is a physical object. In an embodiment, the head-mounted apparatus G is an apparatus in a mixed reality system or an augmented reality system, and the present disclosure is not limited thereto.

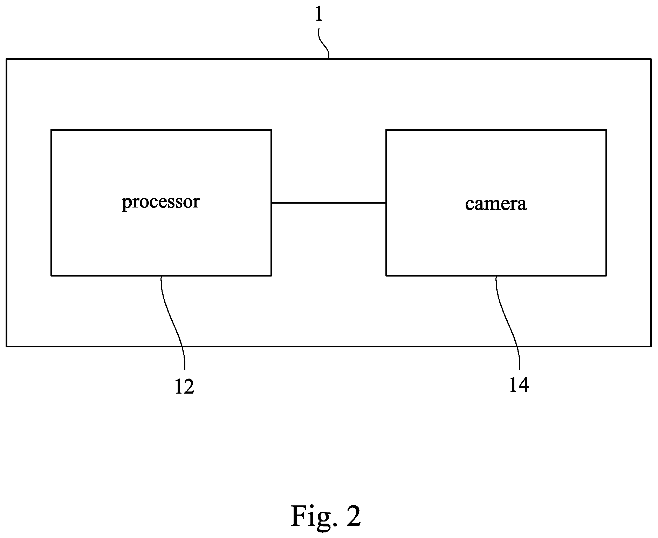

Please refer to Fig. 2, which is a schematic diagram illustrating a posture tracking apparatus 1 according to a first embodiment of the present disclosure. The posture tracking apparatus 1 comprises a processor 12 and a camera 14, wherein the processor 12 is electrically connected to the camera 14. The posture tracking apparatus 1 is configured to determine a head posture of a user. In some embodiments, the posture tracking apparatus 1 is configured in the head-mounted apparatus G shown in Fig. 1 and configured to determine the head posture of the user U. In some embodiments, the posture tracking apparatus 1 is configured in a pair of glasses and configured to determine the head posture of a user.

In some embodiments, the processor 12 comprises a central processing unit (CPU), a graphics processing unit (GPU), a multi-processor, a distributed processing system, an application specific integrated circuit (ASIC), and/or a suitable processing unit.

The camera 14 comprises one or more image capture unit and configured to capture an eye image of a user. In some embodiments, the camera 14 comprises an image capture unit configured in the posture tracking apparatus 1 and shooting towards the user. In some embodiments, the camera 14 comprises a depth image capture unit configured to capture depth images of the user's eye.

It is noted that, the eye image may comprise a single image captured by an image capture unit. Additionally, the eye image may also comprise multiple images captured by image capture units from different viewpoints. The number of the images and the viewpoints are not limited by the present disclosure.

The posture tracking apparatus 1 takes eyes of the user as the medium for shooting the scene outwardly, and the eyes are analogized to the lenses shooting outwardly. In order to achieve the purpose, the posture tracking apparatus 1 calculates postures of the eyes to obtain the viewpoints of the lenses and captures images reflected by the pupils as the scene image captured by the lenses. Furthermore, after obtaining the lens viewpoints and the scene images, the posture tracking apparatus 1 is able to track the head posture of the user accordingly.

Specifically, the processor 12 executes the following operations: calculating an eye posture based on the eye image; capturing a reflected image from the eye image; and calculating a head posture of the user based on the reflected image and the eye posture.

For example, after obtaining the eye posture and the reflected image, the posture tracking apparatus 1 calculates the head posture of the user based on simultaneous localization and mapping (SLAM) algorithm, wherein the head posture is six degrees of freedom (6DoF) data of the user's head.

In some embodiments, the eye image comprises multiple images of the user's eyes shot by the camera 14 in a period of time. Accordingly, the posture tracking apparatus 1 is able to determine the head movement of the user based on the changes of the reflected images in the images, so as to calculate the head posture.

For example, the posture tracking apparatus 1 takes the earliest captured image among the images as a reference and determines the object and/or pattern presented in the reflected image. Accordingly, the posture tracking apparatus 1 determines the position changes of the object and/or pattern in the reflected images from the subsequent images and combines the changes with the eye postures to calculate the head posture.

In some embodiments, since part of the image in the scene and the head posture of the user have been obtained, the posture tracking apparatus 1 may further construct the scene in a three-dimensional space by using the reflected image and the eye posture.

Specifically, the processor 12 is further configured to execute the following operation: constructing a scene image based on the reflected image and the eye posture, wherein the scene image corresponding to a sight direction of the eye posture.

For example, while calculating the head posture based on the simultaneous localization and mapping algorithm, the posture tracking apparatus 1 may also construct a scene image, wherein the scene image comprises the image of the user's surroundings and the relative positions between the image and the user's head in a three-dimensional space.

In some embodiments, in order to increase the accuracy of posture tracking, the posture tracking apparatus 1 further combines inertial data measured by an inertial measurement unit (IMU) to calculate the head posture.

Specifically, the posture tracking apparatus 1 further comprises an inertial measurement unit configured to measure inertial data. Also, the operation of the processor 12 calculating the head posture further comprises: calculating the head posture based on the inertial data corresponding to the eye posture, the reflected image, and the eye posture.

For example, while calculating the head posture based on the simultaneous localization and mapping algorithm, the posture tracking apparatus 1 not only takes the reflected image and the eye posture as the parameters for calculation but also adds inertial data such as acceleration, angular velocity, and orientation for the calculation, wherein the inertial data is measured simultaneously when the eye image is captured.

In some embodiments, while calculating the eye posture, the posture tracking apparatus 1 calculates the positions and the sight directions of the pupils from the eye image and determines the direction the user gazing (i.e., the eye posture) accordingly as the reference for the head posture calculation subsequently.

Specifically, the operation of the processor 12 calculating the eye posture further comprises: calculating a pupil position and a sight direction based on the eye image; and generating the eye posture based on the pupil position and the sight direction.

For example, the posture tracking apparatus 1 calculates the coordinates of the pupils in the image (i.e., the pupil position) and calculates the sight direction vectors of the user (i.e., the sight direction) based on the pupil position. Accordingly, the posture tracking apparatus 1 takes the coordinates and the vectors as the eye posture.

In some embodiments, before calculating the head posture based on the eye posture of the user, the posture tracking apparatus 1 needs to obtain the distance between the eyes of the user as an initial parameter.

Specifically, the operation of the processor 12 calculating the eye posture further comprises: calculating a distance between eyes based on a plurality of eye images for calibration captured from a plurality of different viewpoints; and calculating the eye posture based on the distance between eyes and the eye image.

The posture tracking apparatus 1 may obtain the eye distance of the user in multiple different ways. For example, if the camera 14 comprises multiple image capture units at different viewpoints, the posture tracking apparatus 1 will capture multiple eye images of the user from different viewpoints while the user is wearing the posture tracking apparatus 1. Accordingly, the posture tracking apparatus 1 is able to calculate the eye distance of the user based on the relative positions between the image capture units.

In another embodiment, if the camera 14 comprises a depth image capture unit, the posture tracking apparatus 1 will capture a depth image of the user's eyes while the user is wearing the posture tracking apparatus 1. Accordingly, the posture tracking apparatus 1 is able to calculate the eye distance of the user based on depth information in the depth image.

In another embodiment, if the camera 14 comprises an inertial measurement unit, the posture tracking apparatus 1 will capture the eye image while the user is wearing the posture tracking apparatus 1 and moves the head. Accordingly, the posture tracking apparatus 1 is able to calculate the eye distance of the user based on inertial data measured by the inertial measurement unit and the eye image.

In some embodiments, in order to capture the reflected image, the posture tracking apparatus 1 extracts background eye images from multiple eye images, wherein the background eye images comprise parts of the image not belongs to the image reflected by the pupils, e.g., skin, whites of eyes, pupils. Furthermore, while extracting the reflected image, the posture tracking apparatus 1 removes the background eye image from the eye image to obtain the reflected image.

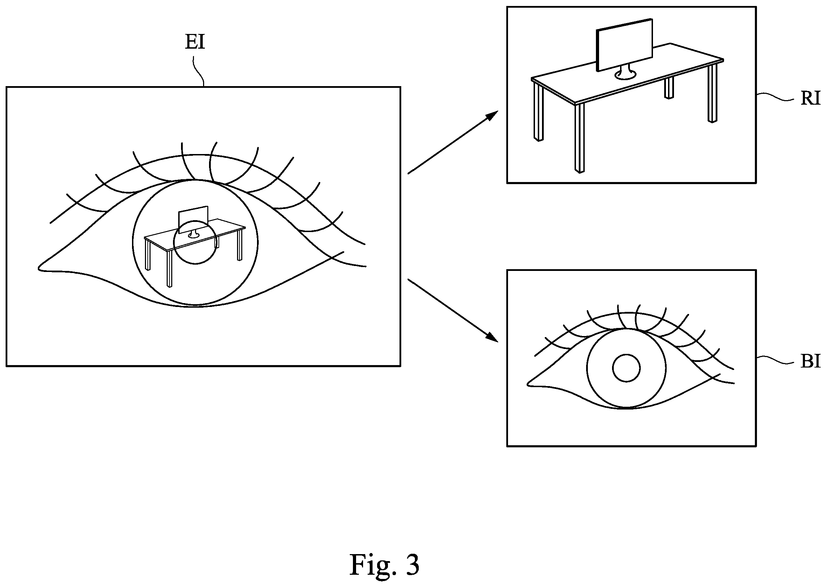

Please refer to Fig. 3, which is a schematic diagram illustrating the posture tracking apparatus 1 capturing a reflected image RI from an eye image EI according to some embodiments of the present disclosure. As shown in the figure, the pupil of the user U in the eye image El reflects the image of the specific object S in the environment E. Through the aforementioned operation, the posture tracking apparatus 1 is able to extract the reflected image RI from the eye image El based on a pre-generated background eye image BI, wherein the reflected image RI presents the image reflected by the pupil.

Specifically, the operation of the processor 12 capturing the reflected image further comprises: generating a background eye image based on a plurality of first reference eye images; and removing the background eye images from the eye image to generate the reflected image.

For example, the first reference eye images may be multiple eye images of the user pre-captured by the camera 14 while the user is wearing, and the processor 12 generates the corresponding background eye image BI accordingly. Subsequently, while calculating the head posture, the posture tracking apparatus 1 is able to generate the reflected image RI based on the background eye image BI then.

It is noted that, the shooting time and the shooting subject of the first reference eye images are not limited by the present disclosure and may be adjusted by the posture tracking apparatus 1 as needed practically. For example, the posture tracking apparatus 1 may capture multiple eye images as the first reference eye images while calculating the head posture or take the eye images of other user as the first reference eye images.

In some embodiments, while calculating the head posture, the posture tracking apparatus 1 gradually calculates the head postures with higher accuracies through multiple iterative operations.

Specifically, the operation of the processor 12 calculating the head posture further comprises a plurality of iterative operations, and each of the iterative operations comprises the following operation: calculating a candidate head posture and a loss based on the reflected image and the eye posture. Also, the operation of the processor 12 calculating the head posture further comprises: selecting the head posture from the candidate head posture corresponding to each of the iterative operations based on the loss corresponding to each of the iterative operations.

For example, the posture tracking apparatus 1 calculates a candidate head posture and a loss corresponding to the candidate head posture in each of the iterative operations by using the simultaneous localization and mapping algorithm. Furthermore, the posture tracking apparatus 1 adjusts parameters based on the gradient direction of the losses and calculates the head posture corresponding to a lower loss (i.e., more accuracy head posture) based on the adjusted parameters in the next iterative operation.

Generally, when a camera shoots images, the different lens designs will lead to different image deformations. Therefore, while calculating the head posture or constructing the scene image, an intrinsic parameter of the camera is needed for calibrating image deformation. On the other hand, since eyeballs are roughly spherical, the image reflected by eyes will also be deformed. Therefore, the corresponding intrinsic parameter are also required for calibrating the reflected image and taken as reference for the subsequent head posture calculation and scene image construction.

The posture tracking apparatus 1 may obtain the intrinsic parameter in different ways. In some embodiments, the posture tracking apparatus 1 sets the intrinsic parameter to an initial value first and then correcting the parameters through the iterative operations by referring to the loss.

Specifically, the operation of the processor 12 calculating the head posture further comprises a plurality of iterative operations, and each of the iterative operations comprises the following operations: calculating a candidate head posture and an optimized parameter based on an initial parameter; and taking the optimized parameter as the initial parameter of the next iterative operation among the iterative operations.

For example, similar with the aforementioned embodiments, while calculating the head posture by using the simultaneous localization and mapping algorithm, the posture tracking apparatus 1 executes the first iterative operation with an initial parameter. After that, in the subsequent iterative operations, the posture tracking apparatus 1 adjusts the intrinsic parameter based on the gradient direction of the losses to obtain more accuracy intrinsic parameter.

In another embodiment, the posture tracking apparatus 1 asks the user for looking a specific image. Since the specific image is known, the posture tracking apparatus 1 is able to calculate the intrinsic parameter by determining the deformation of the specific image in the image reflected by the user's pupils.

Specifically, the operation of the processor 12 calculating the head posture further comprises: obtaining a second reference eye image, wherein the second reference eye image is taken by the camera when the user is looking at a preset image; calculating a deformation parameter based on the second reference eye image and the preset image, wherein the deformation parameter represents a deformation state of the preset image in the second reference eye image; adjusting the reflected image based on the deformation parameter; and calculating the head posture based on the adjusted reflected image and the eye posture.

When the user closes their eyes, the posture tracking apparatus 1 is not able to calculate the head posture based on the eye image. Therefore, in some embodiments, the posture tracking apparatus 1 calculates the head posture based on inertial data instead while the user closes their eyes.

Specifically, the posture tracking apparatus 1 further comprises an inertial measurement unit configured to measure inertial data. The processor 12 is further configured to execute the following operations: determining whether the eye image comprises a valid pupil image; and in response to the eye image does not comprising the valid pupil image, calculating the head posture corresponding to the inertial data based on the inertial data.

For example, similar with the aforementioned embodiments, the posture tracking apparatus 1 also comprises an inertial measurement unit. When determining that the eye image captured by the camera 14 does not comprise pupils, the posture tracking apparatus 1 calculates the head posture based on inertial data measured by the inertial measurement unit instead.

In summary, the posture tracking apparatus 1 provided by the present disclosure calculates the head posture of the user through capturing the eye image of the user. Not only able to reduce the specification requirement and cost, but the posture tracking apparatus 1 also avoids obtaining images the user does not want to be captured. Additionally, the posture tracking apparatus 1 can also construct scene images. Furthermore, the posture tracking apparatus 1 is able to calculate more accuracy head posture through operations comprising initialization, calibration, iterative operations, etc.

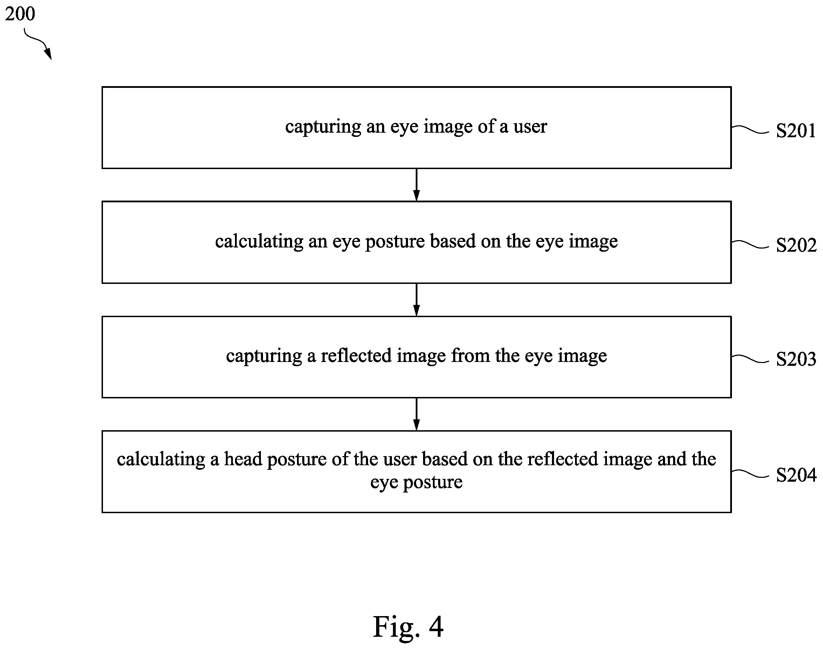

Please refer to Fig. 4, which is a flow diagram illustrating a posture tracking method according to a second embodiment of the present disclosure. The posture tracking method 200 comprises steps S201-S204. The posture tracking method 200 is configured to determine a head posture of a user. The posture tracking method 200 can be executed by an electronic apparatus (e.g., the posture tracking apparatus 1 in the first embodiment).

First, in the step S201, the electronic apparatus captures an eye image of a user.

Next, in the step S202, the electronic apparatus calculates an eye posture based on the eye image.

Next, in the step S203, the electronic apparatus captures a reflected image from the eye image.

Finally, in the step S204, the electronic apparatus calculates a head posture of the user based on the reflected image and the eye posture.

In some embodiments, the step S202 further comprises the electronic apparatus calculating a pupil position and a sight direction based on the eye image; and the electronic apparatus generating the eye posture based on the pupil position and the sight direction.

In some embodiments, the step S202 further comprises the electronic apparatus calculating a distance between eyes based on a plurality of eye images for calibration captured from a plurality of different viewpoints; and the electronic apparatus calculating the eye posture based on the distance between eyes and the eye image.

In some embodiments, the step S203 further comprises the electronic apparatus generating a background eye image based on a plurality of first reference eye images; and the electronic apparatus removing the background eye images from the eye image to generate the reflected image.

In some embodiments, the step S204 further comprises a plurality of iterative operations, and each of the iterative operations comprises the electronic apparatus calculating a candidate head posture and a loss based on the reflected image and the eye posture. Also, the step S204 further comprises the electronic apparatus selecting the head posture from the candidate head posture corresponding to each of the iterative operations based on the loss corresponding to each of the iterative operations.

In some embodiments, the step S204 further comprises a plurality of iterative operations, and each of the iterative operations comprises the electronic apparatus calculating a candidate head posture and an optimized parameter based on an initial parameter; and the electronic apparatus taking the optimized parameter as the initial parameter of the next iterative operation among the iterative operations.

In some embodiments, the step S204 further comprises the electronic apparatus obtaining a second reference eye image, wherein the second reference eye image is taken when the user is looking at a preset image; the electronic apparatus calculating a deformation parameter based on the second reference eye image and the preset image, wherein the deformation parameter represents a deformation state of the preset image in the second reference eye image; the electronic apparatus adjusting the reflected image based on the deformation parameter; and the electronic apparatus calculating the head posture based on the adjusted reflected image and the eye posture.

In some embodiments, the step S204 further comprises the electronic apparatus calculating the head posture based on inertial data corresponding to the eye posture, the reflected image, and the eye posture.

In some embodiments, the posture tracking method 200 further comprises the electronic apparatus constructing a scene image based on the reflected image and the eye posture, wherein the scene image corresponding to a sight direction of the eye posture.

In some embodiments, the posture tracking method 200 further comprises the electronic apparatus determining whether the eye image comprises a valid pupil image; and in response to the eye image does not comprising the valid pupil image, the electronic apparatus calculating the head posture corresponding to inertial data based on the inertial data.

In summary, the posture tracking method 200 provided by the present disclosure calculates the head posture of the user through capturing the eye image of the user. Not only able to reduce the specification requirement and cost, but the posture tracking method 200 also avoids obtaining images the user does not want to be captured. Additionally, the posture tracking method 200 can also construct scene images. Furthermore, the posture tracking method 200 is able to calculate more accuracy head posture through operations comprising initialization, calibration, iterative operations, etc.

Although the present disclosure has been described in considerable detail with reference to certain embodiments thereof, other embodiments are possible. Therefore, the spirit and scope of the appended claims should not be limited to the description of the embodiments contained herein.

It will be apparent to those skilled in the art that various modifications and variations can be made to the structure of the present disclosure without departing from the scope or spirit of the disclosure. In view of the foregoing, it is intended that the present disclosure covers modifications and variations of this disclosure provided they fall within the scope of the following claims.

## Claims

1. A posture tracking apparatus, comprising:
a camera, configured to capture an eye image of a user; and
a processor, electrically connected to the camera, configured to execute the following operation:
calculating an eye posture based on the eye image;
capturing a reflected image from the eye image; and
calculating a head posture of the user based on the reflected image and the eye posture.

2. The posture tracking apparatus of claim 1, wherein the operation of calculating the eye posture further comprises:
calculating a pupil position and a sight direction based on the eye image;
and
generating the eye posture based on the pupil position and the sight direction.

3. The posture tracking apparatus of claim 1, wherein the operation of calculating the eye posture further comprises:
calculating a distance between eyes based on a plurality of eye images for calibration captured from a plurality of different viewpoints; and
calculating the eye posture based on the distance between eyes and the eye image.

4. The posture tracking apparatus of claim 1, wherein the operation of capturing the reflected image further comprises:
generating a background eye image based on a plurality of first reference eye images; and
removing the background eye images from the eye image to generate the reflected image.

5. The posture tracking apparatus of claim 1, wherein the operation of calculating the head posture further comprises a plurality of iterative operations, and each of the iterative operations comprises the following operation:
calculating a candidate head posture and a loss based on the reflected image and the eye posture;
wherein the operation of calculating the head posture further comprises:
selecting the head posture from the candidate head posture corresponding to each of the iterative operations based on the loss corresponding to each of the iterative operations.

6. The posture tracking apparatus of claim 1, wherein the operation of calculating the head posture further comprises a plurality of iterative operations, and each of the iterative operations comprises the following operations:
calculating a candidate head posture and an optimized parameter based on an initial parameter; and
taking the optimized parameter as the initial parameter of the next iterative operation among the iterative operations.

7. The posture tracking apparatus of claim 1, wherein the operation of calculating the head posture further comprises:
obtaining a second reference eye image, wherein the second reference eye image is taken by the camera when the user is looking at a preset image;
calculating a deformation parameter based on the second reference eye image and the preset image, wherein the deformation parameter represents a deformation state of the preset image in the second reference eye image;
adjusting the reflected image based on the deformation parameter; and
calculating the head posture based on the adjusted reflected image and the eye posture.

8. The posture tracking apparatus of claim 1, further comprising:
an inertial measurement unit, configured to measure inertial data;
wherein the operation of calculating the head posture further comprises:
calculating the head posture based on the inertial data corresponding to the eye posture, the reflected image, and the eye posture.

9. The posture tracking apparatus of claim 1, wherein the processor is further configured to execute the following operation:
constructing a scene image based on the reflected image and the eye posture, wherein the scene image corresponding to a sight direction of the eye posture.

10. The posture tracking apparatus of claim 1, further comprising:
an inertial measurement unit, configured to measure inertial data;
wherein the processor is further configured to execute the following operations:
determining whether the eye image comprises a valid pupil image; and
in response to the eye image does not comprising the valid pupil image, calculating the head posture corresponding to the inertial data based on the inertial data.

11. A posture tracking method, being adapted for use in an electronic apparatus, wherein the posture tracking method comprises the following steps:
capturing an eye image of a user;
calculating an eye posture based on the eye image;
capturing a reflected image from the eye image; and
calculating a head posture of the user based on the reflected image and the eye posture.

12. The posture tracking method of claim 11, wherein the step of calculating the eye posture further comprises:
calculating a pupil position and a sight direction based on the eye image;
and
generating the eye posture based on the pupil position and the sight direction.

13. The posture tracking method of claim 11, wherein the step of calculating the eye posture further comprises:
calculating a distance between eyes based on a plurality of eye images for calibration captured from a plurality of different viewpoints; and
calculating the eye posture based on the distance between eyes and the eye image.

14. The posture tracking method of claim 11, wherein the step of capturing the reflected image further comprises:
generating a background eye image based on a plurality of first reference eye images; and
removing the background eye images from the eye image to generate the reflected image.

15. The posture tracking method of claim 11, wherein the step of calculating the head posture further comprises a plurality of iterative operations, and each of the iterative operations comprises the following step:
calculating a candidate head posture and a loss based on the reflected image and the eye posture;
wherein the step of calculating the head posture further comprises:
selecting the head posture from the candidate head posture corresponding to each of the iterative operations based on the loss corresponding to each of the iterative operations.
